(19)

Européisches
Patentamt
European
Patent Office
Office européen
des brevets

(11) **EP 4 116 335 A1**

(12) # EUROPEAN PATENT APPLICATION
published in accordance with Art. 153(4) EPC

(43) Date of publication:
**11.01.2023 Bulletin 2023/02**

(21) Application number: **21764471.5**

(22) Date of filing: **01.03.2021**

(51) International Patent Classification (IPC):
*C08B 37/00* (2006.01)    *A23L 2/52* (2006.01)
*A23L 5/00* (2016.01)    *A23L 33/125* (2016.01)
*A61K 8/73* (2006.01)    *A61K 31/736* (2006.01)
*A61P 1/14* (2006.01)    *C12P 19/04* (2006.01)

(52) Cooperative Patent Classification (CPC):
**A23L 2/52; A23L 5/00; A23L 33/125; A61K 8/73;
A61K 31/736; A61P 1/14; C08B 37/00; C12P 19/04**

(86) International application number:
**PCT/JP2021/007633**

(87) International publication number:
**WO 2021/177208 (10.09.2021 Gazette 2021/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **02.03.2020 JP 2020035288**

(71) Applicant: **Taiyo Kagaku Co., Ltd.
Mie 512-1111 (JP)**

(72) Inventors:
• **TOMINAGA, Etsuko
Yokkaichi-shi, Mie (JP)**

• **SHIMIZU, Kazuo
Suzuka-shi, Mie (JP)**
• **MATSUMIYA, Yoshiki
Tsu-shi, Mie (JP)**
• **SHIMAMURA, Naoki
Tsu-shi, Mie (JP)**
• **YOKAWA, Takeo
Suzuka-shi, Mie (JP)**
• **YONEMURA, Hiroki
Adachi-ku, Tokyo (JP)**
• **NAKAHARA, Yoko
Ota-ku, Tokyo (JP)**

(74) Representative: **Vossius & Partner
Patentanwälte Rechtsanwälte mbB
Siebertstraße 3
81675 München (DE)**

(54) **GALACTOMANNAN DECOMPOSITION PRODUCT**

(57)    A galactomannan degradation product wherein a molecular weight distribution thereof satisfies the following requirements: a molecular weight of 12,000 or more is from 1 to 32%; a molecular weight of from 300 to 3,000 is from 25 to 60%; and a maximum of peak intensity in the molecular weight patterns of high-performance liquid chromatography exists within the range of a molecular weight of from 4,000 to 14,000. The galactomannan degradation product of the present invention can be used in various foodstuff, cosmetics, and the like.

EP 4 116 335 A1

**Description**

TECHNICAL FIELD

The present invention relates to a galactomannan degradation product and foodstuff containing the same.

BACKGROUND ART

Galactomannan is a substance having a comb-shaped branched structure in which the main chain composed of mannose is bound to an $\alpha$-galactosyl group, which has been well utilized conventionally as materials for foodstuff, food additives, feeds, feed additives, medicaments, industrial materials, and the like. In addition, the galactomannan of which molecular weight is reduced by hydrolysis treatment (i.e. galactomannan degradation product) has various physiological properties not found in galactomannan in a polymer state, so that such a galactomannan is being remarked particularly in the recent years (see, for example, Patent Publications 1 to 3).

Patent Publication 1 discloses the technique concerning an agent for improving the intestinal environment, in which a galactomannan degradation product is used as an active ingredient. Patent Publication 2 discloses a technique in which a galactomannan degradation product is used in the production of a composition for growing useful enterobacteria for powdered beverages. Patent Publication 3 discloses a technique concerning an iron absorption promoter in which a galactomannan degradation product is used as an active ingredient.

PRIOR ART REFERENCES

PATENT PUBLICATIONS

Patent Publication 1: Japanese Patent Gazette No. 3008138
Patent Publication 2: Japanese Patent Gazette No. 3441756
Patent Publication 3: Japanese Patent Laid-Open No. Hei-6-247860

SUMMARY OF THE INVENTION

PROBLEMS TO BE SOLVED BY THE INVENTION

However, although the galactomannan degradation products disclosed in Patent Publications 1 to 3 show useful physiological actions to human bodies, there were some cases where the physical properties and the palatability thereof are undesirably greatly changed or some cases where stability is undesirably worsened, depending upon the foodstuff to be added. For example, when used in bread dough, there were some cases where the rise during baking is worsened, and the bread-making properties is lowered, so that the palatability may be lowered in some cases even after baking. In addition, when used in emulsion-type foodstuff such as liquid diets and yogurt beverages, there were some cases where emulsion breaking is caused during storage, so that separation or precipitation may take place in some cases. Therefore, further improvements are desired to make them applicable for these foodstuff.

The present invention relates to the provision of a galactomannan degradation product having small influence on the tastes of foodstuff, having excellent bread-making properties and not greatly undergoing changes in palatability when used in bread dough, and having excellent stability when used in emulsion-type foodstuff.

MEANS TO SOLVE THE PROBLEMS

The present invention relates to the following [1] and [2]:

[1] A galactomannan degradation product wherein a molecular weight distribution thereof satisfies the following requirements:

a molecular weight of 12,000 or more is from 1 to 32%;
a molecular weight of from 300 to 3,000 is from 25 to 60%; and
a maximum of peak intensities in the molecular weight patterns of high-performance liquid chromatography exists within the range of a molecular weight from 4,000 to 14,000.

[2] Foodstuff containing a galactomannan degradation product as defined in [1].

EFFECTS OF THE INVENTION

According to the present invention, a galactomannan degradation product having small influence on the tastes of foodstuff, having excellent bread-making properties and not greatly undergoing changes in palatability when used in bread dough, and having excellent stability when used in emulsion-type foodstuff can be provided.

BRIEF DESCRIPTION OF THE DRAWINGS

[FIG. 1] a graph showing a calibration curve in high-performance liquid chromatography;
[FIG. 2] a graph showing molecular weight patterns of galactomannan degradation products of Example 4 and Comparative Example 1;
[FIG. 3] a graph showing lactic acid contents in feces in the control group and the test groups;
[FIG. 4] a graph showing acetic acid contents in feces in the control group and the test groups;
[FIG. 5] a graph showing propionic acid contents in feces in the control group and the test groups; and
[FIG. 6] a graph showing butyric acid contents in feces in the control group and the test groups.

MODES FOR CARRYING OUT THE INVENTION

As a result of studying the above problems, the present inventors have newly found that the above problems can be solved by using a galactomannan degradation product having a particular molecular weight distribution.
The galactomannan degradation product of the present invention satisfies the following requirements:

a molecular weight of 12,000 or more is from 1 to 32%;
a molecular weight of from 300 to 3,000 is from 25 to 60%; and
a maximum of peak intensities in the molecular weight patterns of high-performance liquid chromatography exists within the range of a molecular weight from 4,000 to 14,000.

Here, the molecular weight of the galactomannan degradation product as used herein is analyzed by the following method. The molecular weight is obtained by using a method for measuring a molecular weight distribution using a high-performance liquid chromatography method (column; TSKgel Super AW (Tosoh Corporation)), and RI (differential refractometer) detector. The details are shown in Examples set forth below. In addition, the amount of the component of the galactomannan degradation product as used herein is expressed as % when the entire peak areas in the above molecular weight distribution are defined as 100%.
The galactomannan refers to a group of polysaccharides in which galactose ($\alpha$-D-galactopyranose) is $\alpha$-(1-6)-bonded to a linear main chain composed of mannose ($\beta$-(1-4)-D-mannopyranose). Galactomannan is one of the substances so-called dietary fibers which are undigested by digestive enzymes owned by human. The galactomannan degradation product of the present invention is one in which this galactomannan is subjected to molecular weight reduction, and the origin thereof can be exemplified by, but not particularly limited to, natural thickening materials, such as guar gum, locust bean gum, tara gum, cassia gum, fenugreek gum, and sesbania gum, the main component of which is galactomannan. Among them, guar gum and locust bean gum can be preferably utilized, and further guar gum is more preferred.
The amount of the component having a molecular weight of 12,000 or more in the galactomannan degradation product of the present invention is 1% or more, preferably 3% or more, and more preferably 5% or more, from the viewpoint of physiological actions, and the amount of the component is 32% or less, preferably 30% or less, more preferably 28% or less, and even more preferably 26% or less, from the viewpoint of bread-making properties and palatability when used in bread dough, and stability when used in emulsion-type foodstuff, and the amount of the component may be within the range of any of these combinations.
The amount of the component having a molecular weight of from 300 to 3,000 in the galactomannan degradation product of the present invention is 25% or more, and preferably 27% or more, from the viewpoint of physiological actions, and the amount of the component is 60% or less, and preferably 50% or less, from the viewpoint of reducing the influences to tastes of the foodstuff, and the amount of the component may be within the range of any of these combinations.
The total amount of the component having a molecular weight of 12,000 or more and the component having a molecular weight of from 300 to 3,000 in the galactomannan degradation product of the present invention is preferably 40% or more, and more preferably 50% or more, and the upper limit can be, for example, 80% or less, and 70% or less, and the total amount may be within the range of any of these combinations.

The amount of the component having a molecular weight of less than 300 in the galactomannan degradation product of the present invention is preferably 15% or less, and more preferably 10% or less, from the viewpoint of reducing the influences on tastes of the foodstuff. The lower limit can be 1% or more, 3% or more, 5% or more, and the like, and the amount of the component may be within the range of any of these combinations. In addition, the amount of the component having a molecular weight exceeding 3,000 and less than 12,000 can be from 20 to 50%.

The galactomannan degradation product of the present invention exists with a molecular weight within the range of 4,000 to 14,000, from the viewpoint of the maximum peak intensities in the molecular weight patterns in the high-performance liquid chromatography, the bread-making properties and palatability when used in bread dough, and stability when used in emulsion-type foodstuff, and it is preferable that the galactomannan degradation product exists with a molecular weight within the range of from 5,000 to 10,000.

When the present galactomannan degradation product is added to foodstuff, it is desired that the exhibition of viscosity is as low as possible. Therefore, the viscosity of the present galactomannan degradation product is preferably 10 mPa•s or less, and more preferably 9 mPa•s or less. The lower limit is not particularly limited, and can be, for example, 6 mPa•s or more, etc., and the viscosity may be within the range of any of the combinations. The viscosity of the galactomannan degradation product as used herein is measured under the following conditions.

Measurement instrument: B-type viscometer,
Rotor No.: BL adaptor,
Rotational speed: 30 rpm,
Measurement time: 60 seconds
Amount of solution: 200 ml in a tall beaker 200ml
Temperature: 20°C, and
Sample: a 15% aqueous solution.

The galactomannan degradation product of the present invention is a substance in which the function of dietary fibers is expected. The dietary fiber content can be measured by a method such as Prosky method (enzyme-weight method), high-performance liquid chromatographic method (enzyme-HPLC method), modified Prosky method, Southgate method, or Englyst method. Among them, the high-performance liquid chromatographic method can analyze even low-molecular water-soluble dietary fibers, the method is suitable for the analysis of the galactomannan degradation product of the present invention. The dietary fiber content when the galactomannan degradation product of the present invention is measured according to the high-performance liquid chromatographic method is 50% or more, preferably 70% or more, and more preferably 80% or more.

In order to obtain the galactomannan degradation product of the present invention, it is inappropriate to not just simply breakdown the sugar chain but the galactomannan degradation product must be sized to a given molecular weight distribution. The method therefor is not particularly limited. For example, the galactomannan degradation product can be produced by a method including degrading galactomannan utilizing hydrolysis or enzymes under acidic or under high-temperature, highpressure conditions in water, and then obtaining a fraction of a particular molecular weight distribution according to a chromatographic method; or degrading a fraction having a molecular weight of a certain level or higher according to oxidation degradation using ozone. Alternatively, the method can be exemplified by a method of obtaining a degradation product in which the reaction conditions in the enzyme reaction are precisely controlled, a method in which a crude galactomannan is selected as a substrate as the galactose side chain to a mannose main chain which is likely to be susceptible to enzyme reaction, or the like. Comprehensively, the method for production with enzymes is preferred for the purposes of sizing the molecular weights.

The enzyme used in the enzyme degradation method may be, and not particularly limited to, any of commercially available ones and naturally derived ones, so long as the enzyme hydrolyzes a mannose linear chain. A β-mannase derived from the bacteria belonging to the genus *Aspergillus,* the bacteria belonging to the genus *Rhizopus*, or the like is preferred.

The method of chromatographic separation is not particularly limited, and a method of classification according to the differences in molecular weight using a porous gel as a carrier is promising. At this time, the fractionation may be carried out with a single-cylinder type chromatography, or a simulated moving bed chromatography by which continuous production can be made industrially is desirably utilized.

It is desired that the galactomannan degradation product of the present invention is previously powdered, from the viewpoints of handling and transport and distribution. The galactomannan degradation product can be hydrolyzed by the method mentioned above, and the hydrolyzed product is then directly dried and powdered with a drier. In order to enhance the quality, after hydrolysis, the hydrolyzed product may be subjected to filtration, treatment with active charcoal or adsorbent, ion-exchanging resin treatment, sterile treatment, or the like, and then dried and powdered. During the powderization, dextrin, saccharides, other dietary fibers, for example, tamarind seed gum or gum arabic, karaya gum, pectin, cellulose, glucomannan, soybean polysaccharides, carrageenan, agar, alginic acid, xanthan gum, gellan gum, agrobacterium succinoglycan, carboxymethyl cellulose, cationic guar gum, or the like may be blended. On the other hand, the galactomannan degradation products in solution states may be preferred in some cases, depending upon the uses and purposes, so that the galactomannan degradation product may be subjected to adjustment of concentrations such as concentrating, as needed, without drying and powdering, which is put into actual use.

In order to more increase the convenience in use, the galactomannan degradation product of the present invention may be, for example, previously mixed and produced into a formulation with the following functional materials and used, or may be allowed to co-exist in foodstuff to which the galactomannan degradation product is finally added. The materials mentioned above can be exemplified by emulsifying agents such as glycerol fatty acid esters, sucrose fatty acid esters, lecithin, antioxidants such as ascorbic acid, tocopherol, flavonoids, and polyphenols, flavors, colorants, and sweeteners.

In addition, the galactomannan degradation product of the present invention is assimilated in intestinal flora so that the exhibiting effects thereby are also expected, and it is expected that the intestinal flora is allowed to grow, to make it useful in the enhancement of the maintenance of health. For this reason, it is useful to ingest together with useful bacteria, or previously mix with useful bacteria to prepare a bacteria formulation. The kinds of bacteria described above can be exemplified by lactic acid bacteria, bifidobacteria, saccharifying bacteria, butyric acid bacteria, and the like.

Since the galactomannan degradation product of the present invention is excellent in bread-making properties and palatability when used in bread dough, and excellent in stability when used in emulsion-type foodstuff, the galactomannan degradation product can be suitably used, but not limited thereto, in breads and emulsion-type foodstuff such as liquid diets, yogurt beverages, coffee beverages, black tea beverages, cocoa beverages, and fruit-and-milk beverages, and can be blended with various foodstuff. For example, the various foodstuff are instant foods such as instant noodles, retort-pouched foods, canned foods, microwave-cooking foods, instant soups and instant miso soups, and freeze-dried foods; beverages such as refreshing beverages, fruit juice beverages, vegetable-based beverages, soymilk beverages, coffee beverages, tea beverages, powdered beverages, concentrated beverages, nutritious beverages, and alcoholic beverages; wheat flour manufactured products such as breads, pastas, uncooked noodles, cake mix, deep frying powders, and panko breadcrumbs; confectioneries such as caramels, candies, chewing gums, chocolates, cookies, biscuits, cakes, pies, snacks, crackers, *wagashi* (Japanese sweets), and dessert confectioneries; seasonings such as sauces, tomato-processed seasonings, flavor seasonings, culinary mix, gravy sauces (*tare*), salad dressings, *tsuyu* (*dashi*-based soy sauce soup base), and roux for curry sauce or stews; fats and oils such as processed fats and oils, butter, margarine, and mayonnaise; dairy manufactured products such as milk beverages, yogurts, lactic acid bacteria beverages, ice creams, and creams; frozen foods; processed marine products such as hams and sausages made of fish meat, and marine pastes; processed livestock products such as livestock hams and sausages; processed agricultural products such as agricultural canned foods, jams and marmalades, pickles, cooked beans, and cereals; supplements in the forms of powders, tablets, capsules, and drinks; nutritious foods such as high-nutritious content foods, liquid diets, concentrated liquid diets, and medical institutional diets, and the like. In addition, the applications can be made to feeds for livestock animals such as bulls and cows, pigs, and poultry; and feeds for household pet animals. The addition method to various foodstuff is not particularly limited, and the galactomannan degradation product can be utilized by previously adding it to raw materials, adding it during the production steps, adding it during cooking, adding it upon eating, or the like. In addition, the galactomannan degradation product of the present invention can be ingested directly in a powder or liquid state, without adding to various foodstuff.

An embodiment of adding a galactomannan degradation product of the present invention to bread is preferably exemplified by an embodiment in which the content of the galactomannan degradation product in the bread dough is adjusted from 1 to 20% by mass.

An embodiment of adding a galactomannan degradation product of the present invention to emulsion-type foodstuff is preferably exemplified by an embodiment in which the content of the galactomannan degradation product in the emulsion-type foodstuff is from 1 to 20% by mass.

In addition, the galactomannan degradation product of the present invention not only can solve the above problems when used in foodstuff, but also can be suitably used in cosmetics. It has been known that moisture-retaining effects and the effects of foam retention are found by adding a galactomannan degradation product to cosmetics. However, the viscosity of cosmetics also increases when a galactomannan degradation product is added, so that there were some problems in feels of use such as being tacky or not being smoothened over the skin, or there were some cases in a pump-type vessel where clogging is caused by the film-forming property of the galactomannan degradation product. For example, the moisture-retaining effects are obtained by adding a galactomannan degradation product of a conventional product to a lotion. On the other hand, there were some cases where the thickening caused by the addition of the galactomannan degradation product undesirably resulted in tackiness. In addition, in W/O type emulsion cosmetics, the tackiness caused by oil and the smoothening over the skin are improved by adding a conventional product galactomannan degradation product. However, on the other hand, there were some cases where the thickening caused by the addition of the galactomannan degradation product undesirably resulted in tackiness. However, it has been newly found that when the galactomannan degradation product of the present invention was added to cosmetics, surprisingly, cosmetics with reduced thickening and with refreshing feels of use, or cosmetics that are less likely to cause clogging in the pump-type vessel can be prepared. Although the mechanisms therefor are not elucidated, it is assumed to be due to the facts that the moisture-retaining effects and the film strength of the foams are increased without increasing the viscosity of the substance by a network structure of a galactomannan degradation product of a certain molecular weight. Therefore, the present invention also provides cosmetics containing a galactomannan degradation product of the present invention. Here, Japanese Patent Laid-Open Hei-4-346908 discloses that a partially degraded product of guar gum having a viscosity as measured with a 1% aqueous solution at 25°C of from 3 to 20 cP is used in cosmetics. However, the viscosity at 25°C when a 1% aqueous solution of a galactomannan degradation product of the present invention is prepared is preferably from 0.1 to 2 mPa•s, more preferably from 0.1 to 1.5 mPa•s, which is different from above.

The cosmetics include, for example, but not particularly limited thereto, skin care products such as milky lotions, creams, lotions, essences, packs, and cleansing agents; makeup products such as lipsticks, foundations, liquid foundations, and makeup pressed powders; hair care products such as hair shampoos, hair rinses, hair treatments, conditioners, hairdyes, and hairdressing; cleansing cosmetics such as cleansing agents, body shampoos, and soaps; further, bathing agents, and the like.

An embodiment of adding a galactomannan degradation product of the present invention to cosmetics is preferably exemplified by an embodiment in which the content of the galactomannan degradation product in the cosmetics is adjusted to from 0.5 to 20% by mass.

As mentioned above, when a galactomannan degradation product of the present invention is used, the foam-retaining property of the cosmetics is improved, and it has been further newly found that foams that have fine textures can be obtained. In addition, as to the effects of improvements in the foam properties mentioned above, it has been newly found that the same is applied in cases where a galactomannan degradation product is added to not only cosmetics but also foodstuff. In other words, when a galactomannan degradation product is added to foodstuff with the expectation of foam-retaining properties, there were some problems such that the smoothness in the feel going down the throat or the external appearance is worsened due to thickening. However, by adding a galactomannan degradation product of the present invention, foams that have an excellent foam-retaining property and very fine textures can be obtained without greatly influencing the smoothness in the feel going down the throat or the external appearance. The foodstuff that are desired to have improvements in foam properties include, for example, beer-taste beverages such as beers, *happo-shu* (bubble water), a new genre, and nonalcoholic beverages; latte-based beverages such as cafe latte, *maccha* late, *hoji-cha* (roasted tea) latte; and foamable foodstuff such as whipped creams, meringues, sponge cakes, soufflés, mousses, and shakes. Therefore, the present invention also provides a method for improving the foam properties of cosmetics or foodstuff by using a galactomannan degradation product of the present invention.

EXAMPLES

The present invention will be specifically described hereinbelow utilizing Examples and Comparative Examples, without intending to limit the present invention thereto. Here, in Examples and Comparative Examples, a guar gum degradation product or a locust bean degradation product was obtained, which may be collectively referred to as a galactomannan degradation product in some cases.

Preparation of Galactomannan Degradation Product

Example 1

A 0.1 N hydrochloric acid was added to 900 g of water to adjust its pH to 3, and 0.15 g of β-mannase derived from the bacteria belonging to the genus *Aspergillus* manufactured by Novo Nordisk Bioindustry, Ltd. and 100 g of a guar gum powder manufactured by Taiyo Kagaku Co., Ltd. were added thereto and mixed, and the enzyme degradation of the guar gum was carried out at 50° to 55°C over 24 hours. After the reaction, the reaction mixture was heated at 90°C for 15 minutes to deactivate the enzyme. The heated mixture was separated by filtration, i.e. suction filtration, to remove insoluble remnants, and this guar gum degradation product was then subjected to a size exclusion chromatography with columns manufactured by Tosoh Corporation: TSK gel SuperOligoPW-N and TSKgel SuperMultipore PW-H, thereby excluding a low-molecular fraction with a molecular weight of about 500 or less and a high-molecular fraction with a molecular weight of about 47,000 or more. In the size exclusion chromatography, pullulan with a molecular weight of 47,300 and maltitriose with a molecular weight of 504 were used as molecular weight markers. This solution was concentrated under reduced pressure with an evaporator manufactured by Yamato, and the concentrate was then dried with a spray-dryer manufactured by OHKAWARA KAKOHKI CO., LTD. to give 54 g of the guar gum degradation product in the form of powder. With respect to the guar gum degradation product obtained, the molecular weight patterns were confirmed by the following method. As a result, the guar gum degradation product had a distribution shown in Table 1.

In addition, a 15% aqueous solution of the guar gum degradation product was prepared, and a viscosity at 20°C was measured with a B-type viscometer manufactured by TOKI SANGYO CO., LTD. In addition, the dietary fiber content was confirmed by the following method. The results are shown in Table 1. Here, a 1% aqueous solution of a guar gum degradation product obtained was prepared, and the viscosity at 25°C was measured with a B-type viscometer manufactured by TOKI SANGYO CO., LTD., and as a result, the viscosity was 1.2 mPa•s.

< Confirmation of Molecular Weight Patterns of Galactomannan Degradation Product >

The molecular weights of the galactomannan degradation products of each of Examples and Comparative Examples were analyzed by using high-performance liquid chromatography. As a sample solution, an aqueous solution of a galactomannan degradation product was prepared, the solid ingredient content concentration of which was 1.0% W/V, and the sample solution was subjected to filtration, and the filtrate was quantitatively analyzed by high-performance liquid chromatography under the following conditions.

HPLC, SHIMADZU, Analysis
Conditions for HPLC:

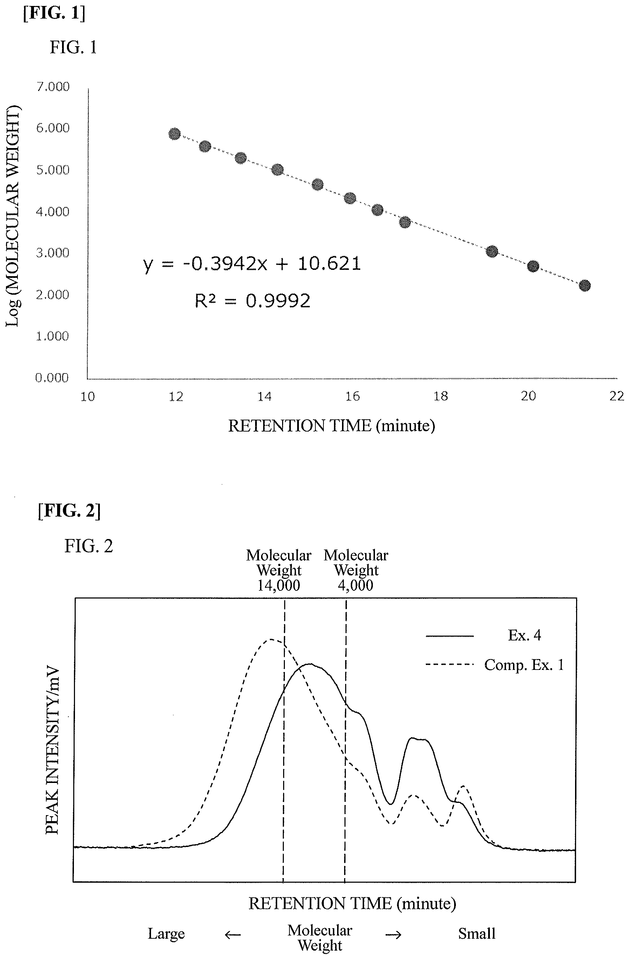

columns: TSKgel SuperAW 4000, Tosoh Corporation, 6 mm*150 mm + TSKgel SuperAW 2500, Tosoh Corporation, 6 mm*150 mm,
detector: RI (differential refractometer),
eluent: water 100%,
flow rate: 0.3 mL/min,
column temperature: 80°C, and
molecular weight markers: pullulans with molecular weights: 5,900, 11,800, 22,800, 47,300, 112,000, 212,000, 404,000, and 788,000 and mannose, maltotriose, and maltoheptaose,

were used to previously obtain a calibration curve showing the relationship between the retention time and the molecular weights as shown in FIG. 1.

< Preparation of Sample Solution for Dietary Fiber Content Analysis >

As a test sample, a guar gum degradation product of Example 1 was taken in a test tube in an amount of 0.1 g on a solid basis, and 5 ml of a 0.08 M phosphate buffer was added thereto to adjust its pH to 6.0. Thereto was added 0.01 ml of a solution of thermostable α-amylase which was heat-resistant α-amylase derived from *Bacillus licheniformis*, manufactured by SIGMA, the test tube was covered with aluminum foil, the contents were reacted in a boiling water bath for 30 minutes while stirring every five minutes, and the reaction mixture was then cooled. A 0.275 M sodium hydroxide solution was added to the liquid reaction mixture obtained to adjust its pH to 7.5. Thereafter, 0.01 ml of a solution of protease derived from *Bacillus licheniformis*, manufactured by SIGMA, attached to a kit was added thereto, the test tube was covered with aluminum foil, and the contents were reacted in a water bath at 60°C for 30 minutes while shaking, and the reaction mixture was then cooled. A 0.325 M hydrochloric acid was added to the protease-treated solution obtained to adjust its pH to 4.3. Thereafter, 0.01 ml of a solution of amyloglucosidase derived from *Aspergillus niger*, manufactured by SIGMA attached to the kit was added thereto, the test tube was covered with aluminum foil, and the contents were reacted in a water bath at 60°C for 30 minutes while shaking, and the reaction mixture was then cooled. Next, desalting was carried out by allowing about 7 ml of the liquid reaction mixture obtained to flow through ion-exchanging resins with SV 1.0, a 1:1 mixture of Amberlite IRA-67, OH form, and Amberlite 200CT, H form, sold by ORGANO Corporation. Further, the desalted mixture was eluted with about 3 times the amount of deionized water, to have a total amount of eluates of about 28 ml. The eluate obtained was concentrated with an evaporator, and the concentrate was filtered with a membrane filter having a pore size of 0.45 μm, and 25 ml of which was quantified with a volumetric flask was used as a sample solution for analysis.

< Conditions for High-Performance Liquid Chromatography >

The sample solution for dietary fiber content analysis obtained above was subjected to high-performance liquid chromatography according to the following conditions.

columns: Two columns of TGKgel G2500PWXL with inner diameter 7.8 mm × length 300 mm, manufactured by Tosoh Corporation, being connected in series;
eluent: deionized water;

sample sugar concentration: 0.8% by mass;
column temperature: 80°C;
flow rate: 0.5 ml/minute;
detection: differential refractometer;
amount injected: 20 μl; and
analyzing time: 50 minutes.

< Calculation of Dietary Fiber Content of Test Samples >

In the chromatogram obtained above, an undigested fraction that remained without being degraded even by the enzyme treatment was defined as dietary fibers. The peak areas of the dietary fibers and glucose that was formed by degradation were each obtained, and the dietary fiber content was calculated from the following formula 1:

$$\text{formula 1:}$$

$$\text{Amount of dietary fibers, mg}$$

$$= \frac{\text{Peak area of dietary fibers}}{\text{Peak area of glucose}} \times \left\{ \begin{array}{l} \text{Mass of glucose, mg*, in} \\ \text{sample solution for analysis} \end{array} \right\}$$

*: Glucose concentration (mg/ml) in sample solution for analysis × 25 mL
using the amount of glucose in the sample solution for analysis which was separately quantified according to the conventional glucose oxidase method. Further, the dietary fiber content of the test sample was calculated from the following formula 2.

$$\text{formula 2:}$$

$$\text{Dietary fiber content, \% by mass}$$

$$= \frac{\text{Amount of dietary fiber, mg, of the test sample}}{\text{Mass of solid content, mg, of the test sample}} \times 100$$

Example 2

A 0.1 N hydrochloric acid was added to 20 kg of water to adjust its pH to 4, and 3 g of β-mannase derived from the bacteria belonging to the genus *Aspergillus* manufactured by Novo Nordisk Bioindustry, Ltd. and 2 kg of a locust bean gum powder manufactured by Taiyo Kagaku Co., Ltd. were added thereto and mixed, and the enzyme degradation of the locust bean gum was carried out at 75°C over 24 hours. After the reaction, the reaction mixture was heated at 90°C for 30 minutes to deactivate the enzyme. The heated mixture was separated by filtration, i.e. suction filtration, to remove insoluble remnants, the transparent solution obtained was concentrated under a reduced pressure with an evaporator manufactured by Yamato, the amount of solid content of which was 20% by mass, and the concentrate was then dried with a spray-dryer manufactured by OHKAWARA KAKOHKI CO., LTD. to give 1.7 kg of the locust bean gum degradation product in the form of powder. One kilogram of the locust bean gum degradation product obtained was dissolved in 20 kg of water, and the solution was subjected to a treatment with ozone nanofoam water Foamest O$^3$ at an ozone supplying rate of 200 mg/h and a rate of ozone water generated of 5 L/min for 15 minutes. Next, the treated solution obtained was dried with a spray-dryer manufactured by OHKAWARA KAKOHKI CO., LTD. to give 940 g of the locust bean gum degradation product in the form of powder. The molecular weight patterns, the viscosity, and the dietary fiber content were confirmed in the same manner as in Example 1. The results are shown in Table 1.

Example 3

A 0.1 N hydrochloric acid was added to 180 kg of water to adjust its pH to 4, and 30 g of β-mannase derived from the bacteria belonging to the genus *Aspergillus* manufactured by Novo Nordisk Bioindustry, Ltd. and 20 kg of a guar gum powder manufactured by Taiyo Kagaku Co., Ltd. were added thereto and mixed, and the enzyme degradation of the guar gum was carried out at 75°C over 24 hours. After the reaction, the reaction mixture was heated at 90°C for 30 minutes to deactivate the enzyme. The heated mixture was separated by filtration, i.e. suction filtration, to remove insoluble remnants, and this guar gum degradation product was then subjected to a size exclusion chromatography with columns manufactured by Tosoh Corporation: TSK gel SuperOligoPW and TSKgel SuperMultipore PW-M, thereby excluding a low-molecular fraction with a molecular weight of about 200 or less and a high-molecular fraction with a molecular weight of about 20,000 or more. In the size exclusion chromatography, mannose with a molecular weight of 180 and pullulan with a molecular weight of 22,800 were used as molecular weight markers. This solution was concentrated under reduced pressure with an evaporator manufactured by Yamato, and the concentrate was then dried with a spray-dryer manufactured by OHKAWARA KAKOHKI CO., LTD. to give 10 kg of the guar gum degradation product in the form of powder. The molecular weight patterns, the viscosity, and the dietary fiber content were confirmed in the same manner as in Example 1. The results are shown in Table 1.

Example 4

A 0.1 N hydrochloric acid was added to 900 g of water to adjust its pH to 4, and 0.15 g of β-mannase derived from the bacteria belonging to the genus *Aspergillus* manufactured by Novo Nordisk Bioindustry, Ltd. and 100 g of a guar gum powder manufactured by Taiyo Kagaku Co., Ltd. were added thereto and mixed, and the enzyme degradation of the guar gum was carried out at 75°C over 24 hours. After the reaction, the reaction mixture was heated at 90°C for 15 minutes to deactivate the enzyme. The heated mixture was separated by filtration, i.e. suction filtration, to remove insoluble remnants, and this guar gum degradation product was then subjected to a size exclusion chromatography with columns manufactured by Tosoh Corporation: TSK gel SuperOligoPW and TSKgel SuperMultipore PW-M, thereby excluding a low-molecular fraction with a molecular weight of about 200 or less and a high-molecular fraction with a molecular weight of about 20,000 or more. In the size exclusion chromatography, mannose with a molecular weight of 180 and pullulan with a molecular weight of 22,800 were used as molecular weight markers. This solution was concentrated under reduced pressure with an evaporator manufactured by Yamato, and the concentrate was then dried with a spray-dryer manufactured by OHKAWARA KAKOHKI CO., LTD. to give 48 g of the guar gum degradation product in the form of powder. The molecular weight patterns, the viscosity, and the dietary fiber content were confirmed in the same manner as in Example 1. The results are shown in Table 1 and FIG. 2.

Example 5

One kilogram of a guar gum degradation product fractionated in Example 3 was dissolved in 20 kg of water, and the solution was subjected to a treatment with ozone nanofoam water Foamest $O^3$ for 120 minutes at an ozone supplying rate of 50 mg/h and a rate of ozone water generated of 3 L/min. Next, the treated solution obtained was dried with a spray-dryer manufactured by OHKAWARA KAKOHKI CO., LTD. to give 900 g of the guar gum degradation product in the form of powder. The molecular weight patterns, the viscosity, and the dietary fiber content were confirmed in the same manner as in Example 1. The results are shown in Table 1.

Comparative Example 1

A 0.1 N hydrochloric acid was added to 900 g of water to adjust its pH to 4, and 0.15 g of β-mannase derived from the bacteria belonging to the genus *Aspergillus* manufactured by Novo Nordisk Bioindustry, Ltd. and 100 g of a guar gum powder manufactured by Taiyo Kagaku Co., Ltd. were added thereto and mixed, and the enzyme degradation of the guar gum was carried out at 75°C over 24 hours. After the reaction, the reaction mixture was heated at 90°C for 15 minutes to deactivate the enzyme. The heated mixture was separated by filtration, i.e. suction filtration, to remove insoluble remnants, the transparent solution obtained was concentrated under reduced pressure with an evaporator manufactured by Yamato, and the concentrate was then dried with a spray-dryer manufactured by OHKAWARA KAKOHKI CO., LTD. to give 68 g of the guar gum degradation product in the form of powder. The molecular weight patterns, the viscosity, and the dietary fiber content were confirmed in the same manner as in Example 1. The results are shown in Table 1 and FIG. 2.

Comparative Example 2

A 0.1 N hydrochloric acid was added to 900 g of water to adjust its pH to 3, and 0.15 g of β-mannase derived from the bacteria belonging to the genus *Aspergillus* manufactured by Novo Nordisk Bioindustry, Ltd. and 100 g of a guar gum powder manufactured by Taiyo Kagaku Co., Ltd. were added thereto and mixed, and the enzyme degradation of the guar gum was carried out at 50° to 55°C over 24 hours. After the reaction, the reaction mixture was heated at 90°C for 15 minutes to deactivate the enzyme. The heated mixture was separated by filtration, i.e. suction filtration, to remove insoluble remnants, and this guar gum degradation product was then subjected to a size exclusion chromatography with a column manufactured by Tosoh Corporation: TSK gel SuperOligoPW, thereby excluding a low-molecular fraction with a molecular weight of about 200 or less. In the size exclusion chromatography, mannose with a molecular weight of 180 was used as a molecular weight marker. This solution was concentrated under reduced pressure with an evaporator manufactured by Yamato, and the concentrate was then dried with a spray-dryer manufactured by OHKAWARA KAKOHKI CO., LTD. to give 54 g of the guar gum degradation product in the form of powder. The molecular weight patterns, the viscosity, and the dietary fiber content were confirmed in the same manner as in Example 1. The results are shown in Table 1.

Comparative Example 3

A 0.1 N hydrochloric acid was added to 900 g of water to adjust its pH to 4, and 0.15 g of β-mannase derived from the bacteria belonging to the genus *Aspergillus* manufactured by Novo Nordisk Bioindustry, Ltd. and 100 g of a guar gum powder manufactured by Taiyo Kagaku Co., Ltd. were added thereto and mixed, and the enzyme degradation of the guar gum was carried out at 50° to 55°C over 24 hours. After the reaction, the reaction mixture was heated at 90°C for 15 minutes to deactivate the enzyme. The heated mixture was separated by filtration, i.e. suction filtration, to remove insoluble remnants, and this guar gum degradation product was then subjected to a size exclusion chromatography with columns manufactured by Tosoh Corporation: TSK gel SuperOligoPW-N and TSK gel SuperMultipore PW-H and PW-M, PW-N, thereby excluding a high-molecular fraction with a molecular weight of about 11,000 or more. In the size exclusion chromatography, pullulan with a molecular weight of 11,800 was used as a molecular weight marker. This solution was concentrated under a reduced pressure with an evaporator manufactured by Yamato, the amount of solid content of which was 19% by mass, and the concentrate was then dried with a spray-dryer manufactured by OHKAWARA KAKOHKI CO., LTD. to give 58 g of the guar gum degradation product in the form of powder. The molecular weight patterns, the viscosity, and the dietary fiber content were confirmed in the same manner as in Example 1. The results are shown in Table 1.

Lemon Beverages: Influences of Sweetness by Addition of Galactomannan Degradation Product

A lemon beverage containing 3.0% by mass of a galactomannan degradation product of each of Examples 1 to 5 and Comparative Examples 1 to 3 (pH of 3, citric acid: 0.08% by mass, lemon flavor manufactured by T. HASEGAWA CO., LTD.: 0.1% by mass, the pH adjusted with trisodium citrate) was prepared.

< Evaluation of Sweetness >

The sensory evaluation regarding the sweetness of a lemon beverage prepared by adding 3.0% by mass of a galactomannan degradation product of each of Examples 1 to 5 and Comparative Examples 1 to 3 (pH of 3, citric acid: 0.08% by mass, lemon flavor manufactured by T. HASEGAWA CO., LTD.: 0.1% by mass, the pH adjusted with trisodium citrate) was made in 5-ranks by seven trained panelists by the following criteria, and an average score of a total point was calculated. The results are shown in Table 1. The sweetness by the galactomannan degradation product was subjected to a sensory evaluation comprehensively in sugar sweetness and unpleasant lingering taste (lingering sweetness, or the like), and the evaluation was made by defining a non-added product as a rank 5.

(Evaluation Criteria)

1: Tastes strong sweetness as compared to the non-added product.
2: Tastes sweetness as compared to the non-added product.
3: Tastes some sweetness as compared to the non-added product.
4: Tastes slight sweetness as compared to the non-added product.
5: No sweetness, the same taste as the non-added product.

Making of Bread According to Sponge Dough Method

In a mixer-ball were supplied 70 parts by mass of bread flour, 1.2 parts by mass of dry yeast, 0.1 parts by mass of yeast food, and 42 parts by mass of water, and the contents were mixed at a low speed for 4 minutes, and a moderate speed for 2 minutes, to give a *nakadane* (sponge) dough. The dough was allowed to rise at 27°C for 4 hours, 30 parts by mass of bread flour, 8 parts by mass of sugar, 10 parts by mass of heavy cream, 2 parts by mass of salt, 0.3 parts by mass of malt syrup, and 23 parts by mass of water were then added thereto, and the contents were mixed at a low speed for 4 minutes, and a high speed for 3 minutes. Thereafter, 4 parts by mass of butter and 2 parts by mass of enzyme-containing shortening were supplied thereto. Thereto was added 6.7 parts by mass of a galactomannan degradation product of each of Examples 1 to 5 and Comparative Examples 1 and 2, and the contents were then additionally mixed at a low speed for 1 minute, and a high speed for 3 minutes, to give a main kneaded dough. The dough was allowed to rise at 27°C for 30 minutes, and weighed out and divided so that each weighed 215 g per dough. The divided dough was rolled into a ball, and the ball-shaped dough was allowed to sit for additional 20 minutes. The bread-making properties of the bread dough obtained was evaluated by the following criteria. Next, the dough was molded, placed in a case for one loaf, and subjected to final fermentation in a fermentation tank at 38°C and humidity of 85% for 100 minutes. Subsequently, a cover was placed over the fermented dough and baked in an oven with an upper fire set at 190°C and a lower fire at 210°C for 20 minutes, to give bread. The palatability of the bread obtained was evaluated by the following criteria. The evaluation results are shown in Table 1.

< Evaluation of Bread-Making Properties of Dough >

The state of the bread dough obtained above was observed, and the bread-making properties was evaluated. The evaluation criteria during the evaluation are as follows, where a dough with no addition of the galactomannan degradation product was evaluated as 5.

(Evaluation Criteria)

5: The dough is without tackiness and with tension and stretch, so that the dough is in a highly excellent state, and the dough has highly excellent bread-making properties.
4: The dough is with slight tackiness but with tension and stretch, so that the dough is in an excellent state, and the dough has excellent bread-making properties.
3: The dough is with tackiness, with slightly deficient tension but with stretch, so that there are no problems in a state of the dough, and there are no problems in the bread-making properties of the dough.
2: The dough has slightly stronger tackiness, slightly lowered tension and lacks a chewy texture so that the dough is slightly worsened in its state, and the bread-making properties of the dough is slightly worsened.
1: The dough has stronger tackiness and is without tension or chewy texture, and it is difficult to roll into a ball or to mold so that the state of the dough is worsened, and the bread-making properties of the dough is worsened.

< Evaluation of Palatability >

Ten trained panelists were asked to eat the bread obtained above, to carry out a sensory evaluation, and the scores were averaged to obtain the evaluation results. The evaluation criteria during the evaluation are as follows, and the evaluation of the bread without the addition of the galactomannan degradation product, i.e., conventional bread, was 4.8.

(Evaluation Criteria)

5: The softness is the same or better than the conventional bread, without sandy tastes, and is highly preferred.
4: The softness is the same or better than the conventional bread, without hardly any sandy tastes, and being even more preferred.
3: The softness is at a level without problems, without hardly any sandy tastes, and is preferred.
2: At least one of softness and sandy taste is slightly worse than the conventional bread, and not at a satisfactory level.
1: At least one of softness and sandy taste is clearly worse than the conventional bread, and at a level having problems.

< Evaluation of Stability in Liquid Diets >

To a commercially available concentrated liquid diet Egao Kurabu (Smile Club), FoodCare Co., Ltd. was added each of the galactomannan degradation products of Examples 1 to 5 and Comparative Examples 1 and 2 so as to have a concentration of 5 g/100 mL with stirring. The mixture was then packed in a bottle, and subjected to retort sterilization at 121°C for 10 minutes. After cooling, the packed mixture was stored at 37°C, and the stability after one week was compared with the concentrated liquid diet without the addition of the galactomannan degradation product and evaluated by the following evaluation criteria. The evaluation methods were such that the floating appearances of a ring were confirmed daily, and the presence or absence of the precipitates at the bottom of the bottle was confirmed visually once every two days. The results are shown in Table 1.

(Evaluation Criteria)

◎: being highly homogeneous;
○: being homogeneous; and
×: being separated.

< Evaluation of Stability in Yogurt Beverages >

To a commercially available yogurt beverage Bulgaria Drinkable Yogurt, Meiji Co., Ltd. was added each of the galactomannan degradation products of Examples 1 to 5 and Comparative Examples 1 and 2 so as to have a concentration of 5 g/100 mL with stirring. The mixture was then packed in a bottle, and subjected to retort sterilization at 121°C for 10 minutes. After cooling, the packed mixture was stored at 4°C, and the stability after one week was compared with the concentrated liquid diet without the addition of the galactomannan degradation product and evaluated by the following evaluation criteria. The evaluation methods were such that the separation and water release were confirmed daily, and the presence or absence of the precipitates at the bottom of the bottle was confirmed visually once every two days. The results are shown in Table 1.

(Evaluation Criteria)

◎: being highly homogeneous;

○: being homogeneous; and

×: being separated.

[Table 1]

Table 1

| | Comp. Ex. 1 | Comp. Ex. 2 | Ex. 1 | Ex. 2 | Ex. 3 | Ex. 4 | Ex. 5 | Comp. Ex. 3 |
|---|---|---|---|---|---|---|---|---|
| Molecular weight of 12,000 or more | 50.8 | 40.3 | 31.4 | 29.0 | 25.5 | 23.7 | 4.9 | 8.4 |
| Molecular weight of from 300 to 3,000 | 15.7 | 18.2 | 28.3 | 26.0 | 32.5 | 31.1 | 48.7 | 66.9 |
| Molecular weight of less than 300 | 7.8 | 9.3 | 8.8 | 9.1 | 9.8 | 9.4 | 11.3 | 17.4 |
| Maximum of molecular weight peak | 21,500 | 15,200 | 13,800 | 11,600 | 8,110 | 8,070 | 4,770 | 3,660 |
| Viscosity, mPa•s | 27.0 | 11.9 | 9.8 | 9.1 | 8.0 | 7.9 | 6.1 | 5.8 |
| Dietary fiber content, % | 85.9 | 85.7 | 85.2 | 84.9 | 84.6 | 84.5 | 59.7 | 36.3 |
| Sweetness | 3.9 | 4.2 | 3.9 | 4.0 | 4.1 | 3.8 | 3.0 | 1.7 |

(continued)

| | Comp. Ex. 1 | Comp. Ex. 2 | Ex. 1 | Ex. 2 | Ex. 3 | Ex. 4 | Ex. 5 | Comp. Ex. 3 |
|---|---|---|---|---|---|---|---|---|
| Bread-making properties of dough | 1 | 2 | 4 | 4 | 5 | 5 | 5 | - |
| Palatability | 1.8 | 2.1 | 3.4 | 3.6 | 4.8 | 4.7 | 4.8 | - |
| Stability in liquid diet | × | × | ○ | ○ | ◎ | ◎ | ◎ | - |
| Stability in yogurt beverage | × | × | ○ | ○ | ◎ | ◎ | ◎ | - |

From Table 1, each of the galactomannan degradation products of Examples 1 to 5 had more controlled sweetness as compared to that of Comparative Example 3, and the influences on the taste of foodstuff would be small. Here, Comparative Example 3 had such strong sweetness that made it unsuitable to be applied to foods, so that the evaluation tests other than the evaluation of sweetness were not conducted. The bread dough in which each of the galactomannan degradation products of Examples 1 to 5 was added had more excellent bread-making properties as compared to the bread dough in which the galactomannan degradation product of Comparative Example 1 or 2 was added so that the palatability when eaten as bread was also excellent. In addition, each of the galactomannan degradation products of Examples 1 to 5 was homogeneous and stable without undergoing separation during the storage period, when added to a liquid diet or a yogurt beverage.

< Physiological Actions (Animal Tests) >

Experimental Method

Test Animals and Feeds:

Rats used in the test were 30 four-week-old Sprague-Dawley (SD) lineage male rats purchased from Japan SLC, Inc. The rats were bred in a stainless steel mesh cage under the conditions of room temperature of $25° \pm 2°C$, humidity of from 40 to 60%, and 12-hour bright-dark cycles. After pre-breeding for one week, the rats were divided into five groups so that a significant difference in body weights of the rats cannot be initially found. During the test period, the body weight and the amount of ingested feeds were measured once a week. Feces before the administration of test feeds, and after one week and four weeks from the administration were collected, and the organic acid contents in feces were measured. The control group was fed with a low dietary fiber diet containing 8% cellulose, and the test group was fed with feeds in which 5% each of a guar gum degradation product (Example 4, and Comparative Examples 1 and 2) or sparingly digestible dextrin manufactured by Matsutani Chemical Industry Co., Ltd., under the trade name of PINE FIBER was added to the low dietary fiber diet. The rats were allowed to take the feeds and water *ad libitum*, and the body weights and the amount of ingested feeds were daily measured. The handling of the test animals was in compliance with the guidelines of the Ethics Committee.

Measurement of Organic Acid Contents in Feces

The organic acid contents in feces were measured by high-performance liquid chromatography. Specifically, to 0.8 g of feces were added 1 ml of a 0.2 w/v % levulinic acid solution (internal standard solution) and 9 ml of distilled water, and the mixture was subjected to ultrasonication treatment for 2 minutes. The suspension was heated at 100°C for 5 minutes, then centrifuged at 3,000 rpm for 15 minutes, ethanol was added to the supernatant, and the mixture was centrifuged again at 3,000 rpm for 15 minutes. The supernatant was concentrated under reduced pressure, a 10% methanol was added to the concentrate to dissolve, the liquid mixture was then centrifuged at 15,000 rpm for 5 minutes, and the supernatant was filtered with a 0.45 $\mu$m membrane filter. This filtrate was subjected to a measurement by a method for the detection of electric conductivity using high-performance liquid chromatography. In the analysis, the separation was carried out using two columns of sim-pack SCR-102H, SHIMADZU CORPORATION, 8.0 mm I.D. $\times$ 300 mm, connected in series (mobile phase: 5 mM p-toluenesulfonic acid solution, flow rate: 0.8 ml/min, column temperature: 45°C), and detected with an electric conductivity detector (test solution: 20 mM Bis-Tris aqueous solution containing a 5 mM p-toluenesulfonic acid solution and 100 $\mu$M EDTA, flow rate: 0.8 ml/min). As the standard substances, lactic acid and acetic acid, propionic acid, and butyric acid were used.

Experiment Results

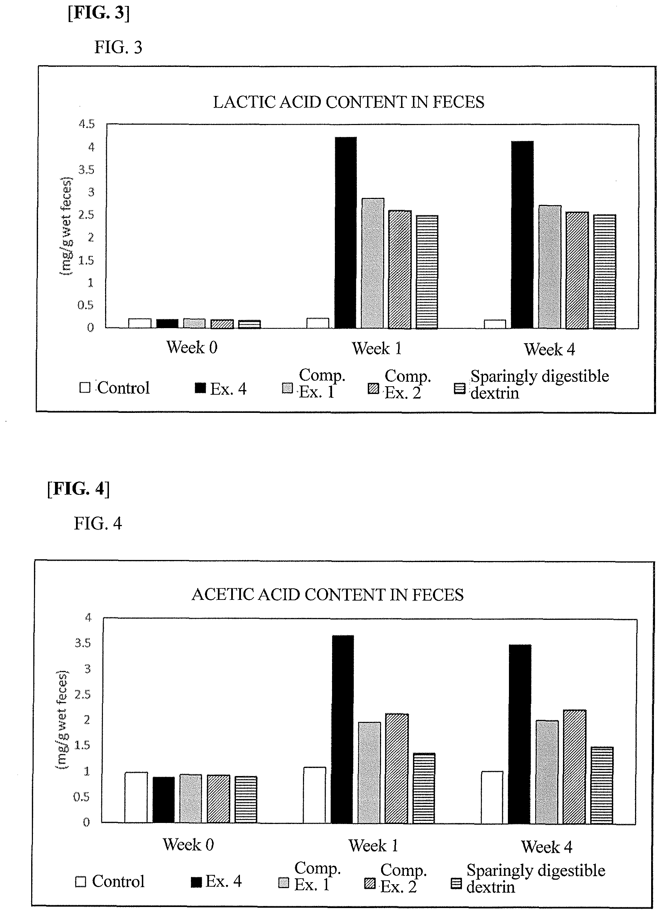

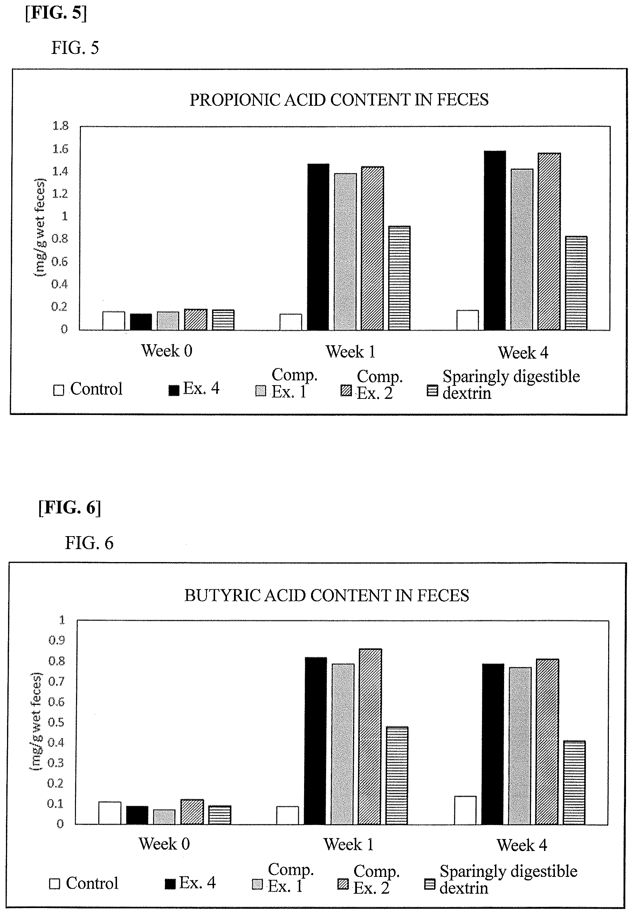

There were no differences between the control group and the test group in the body weight changes and the amount of ingested feed during the test period. The organic acid contents in feces during the test period are shown in FIGs. 3 to 6. During the test period, the test group had an increase in the organic acid contents in feces as compared to the control group. In other words, this means that the intestinal environment was maintained on an acidic side, so that it can be seen that the test group has physiological action of regulating the intestinal environment. In particular, in the group in which a guar gum degradation product of Example 4 was ingested, the amounts of lactic acid and acetic acid produced were more increased than in the group in which a guar gum degradation product shown in Comparative Example 1 or 2 or sparingly digestible dextrin was ingested. In the propionic acid content and the butyric acid content in feces, the contents were of the same levels in the group in which the guar gum degradation product of Example 4 and Comparative Examples 1 and 2, but an increase in the levels was confirmed than the group in which sparingly digestible dextrin was ingested.

Test Example 1 - Lotions

Lotions 1 to 8 were prepared with a composition as listed in Table 2 using each of the galactomannan degradation products prepared in Examples 1 to 5 and Comparative Examples 1 to 3. In addition, the same procedures as above were carried out except that the galactomannan degradation product was not used to prepare Lotion 9. The sensory evaluation was carried out by ten panelists, and questionnaires were conducted. The panelists wrote down the judgment of the sensory evaluation on skin textures on the response sheet in a 6-rank evaluation, in which the numerical figure when the effects felt the weakest was defined as 1, and the numerical figure when the effects felt the strongest was defined as 6. The value in which the numerical figures responded by the ten individuals were averaged was regarded as additive effects of Examples and Comparative Examples. The judgments were made such that it was recognized to have some effects when the numerical figures were from 4 to 6, and it was recognized not to have effects when the numerical figures were less than 4. The items of the sensory evaluation were 3 items, "smooth skin texture after application," "moisturized skin texture after application," and "being absorbed well into skin after application." The results are shown in Table 3.

[Table 2]

Table 2: Lotions

| Indicated name | Blending amount, % by mass |
| --- | --- |
| Pentylene glycol | 1.00 |
| DPG | 2.00 |
| BG | 2.00 |
| Glycerol | 1.50 |
| Citric acid | 0.03 |
| Sodium citrate | 0.08 |
| Betaine | 0.05 |
| Galactomannan degradation product (Examples, Comparative Examples) | 2.00 |
| Water | Balance |
| Methylparaben | 0.02 |
| Total | 100.00 |

[Table 3]

Table 3

| Lotion | Composition used | Results | | |
|---|---|---|---|---|
| | | Smooth skin texture after application | Moisturized skin texture after application | Being absorbed well into skin after application |
| 1 | Comp. Ex. 1 | 1.2 | 2.3 | 1.2 |
| 2 | Comp. Ex. 2 | 1.4 | 2.9 | 1.6 |
| 3 | Ex. 1 | 4.4 | 4.4 | 4.9 |
| 4 | Ex. 2 | 4.6 | 5.3 | 5.3 |
| 5 | Ex. 3 | 5.1 | 5.5 | 5.2 |
| 6 | Ex. 4 | 5.4 | 5.6 | 5.1 |
| 7 | Ex. 5 | 5.8 | 5.8 | 5.2 |
| 8 | Comp. Ex. 3 | 4.0 | 1.2 | 2.9 |
| 9 | Without addition | 4.9 | 1.0 | 2.6 |

As is clear from the results of Table 3, when the inventive products were used as cosmetics, it can be seen that the skin texture after the application is smooth, that a feel of moisturizing skin is obtained, and that the inventive products are favorably absorbed to skin.

Test Example 2 - Body Wash

Each of Body washes 1 to 4 was prepared with a composition as listed in Table 4 using each of the galactomannan degradation products prepared in Examples 4 and 5 and Comparative Examples 1 and 3. In addition, Body wash 5 was prepared in the same manner as above except that the galactomannan degradation product was not used. The viscosity of the body washes prepared at 25°C was measured with a B type viscometer manufactured by TOKI SANGYO CO., LTD. The sensory evaluation was carried out by 10 panelists, and questionnaires were conducted on "smooth skin texture without being slimy," "fineness of foams," and "foam-retention property." The panelists were asked to write down the judgments on the sensory evaluations on skin textures on the response sheets in a 6-rank evaluation, in which the numerical figure when the effects felt the weakest was defined as 1, and the numerical figure when the effects felt the strongest was defined as 6. The value in which the numerical figures responded by the ten individuals were averaged was regarded as additive effects of Examples and Comparative Examples. The judgments were made such that it was recognized to have some effects when the numerical figures were from 4 to 6, and it was recognized not to have effects when the numerical figures are less than 4. In addition, the prepared body wash was diluted in one-half with water, and filled in a pump foamer, a vessel capable of ejecting foams by pressing a nozzle portion, and ejected therefrom, and the foam was then stored at 50°C for one month, and the clogging of the pump was evaluated by the following evaluation criteria. The results are shown in Table 5.

< Evaluation Criteria >

X: the ejection nozzles being clogged, making it impossible to eject the body wash;
△: the amount ejected being small, the body wash ejected in a liquid state; and
○: no changes in the amount ejected and the state of foams from those before storage.

[Table 4]

Table 4: Body wash

| Indicated name | Blending amount, % by mass |
|---|---|
| Polyglycerol laurate-10 | 4.0 |
| Sodium laurylsulfate | 26.0 |
| Lauryl hydroxysultaine | 7.5 |
| Cocamide DEA | 4.0 |
| BG | 6.0 |
| Citric acid | 1.3 |
| Galactomannan degradation product (Examples, Comparative Examples) | 5.0 |
| Water | Balance |
| Total | 100.00 |

[Table 5]

Table 5

| Body wash | Product used | Viscosity, mPa•s | Smooth skin texture without being slimy | Fineness of foams | Foam-retention property | Clogging of pump at 50°C, one month |
|---|---|---|---|---|---|---|
| | | | Results | | | |
| 1 | Comp.Ex. 1 | 498 | 1.2 | 2.1 | 2.5 | × |
| 2 | Ex. 4 | 156 | 5.8 | 5.1 | 5.2 | ○ |
| 3 | Ex. 5 | 142 | 5.6 | 5.8 | 5.7 | ○ |
| 4 | Comp.Ex. 3 | 135 | 4.2 | 1.1 | 1.5 | △ |
| 5 | Without addition | 129 | 5.8 | 1.1 | 1.1 | ○ |

As is clear from the results of Table 5, when the inventive products were used in a body wash, it can be seen that the texture of foams is fine, that the foam-retaining property is excellent, that there is no sliminess as the feel of use, and that a moisturized feel is obtained after cleansing, even though the viscosity of the body wash itself is low. Also, the clogging of the pump was not observed.

Test Example 3 - Emulsion type Foundation

Each of Emulsion type foundations 1 to 3 was prepared with a composition as listed in Table 6 using each of the galactomannan degradation products prepared in Examples 2 and 4 and Comparative Examples 1 and 3. In addition, Emulsion type foundation 4 was prepared in the same manner as above except that the galactomannan degradation product was not used. The sensory evaluation was carried out by 10 panelists, and questionnaires were conducted on feel of use. The panelists were asked to write down the judgments on the sensory evaluations on skin textures on the response sheets in a 6-rank evaluation, in which the numerical figure when the effects felt the weakest was defined as 1, and the numerical figure when the effects felt the strongest was defined as 6. The value in which the numerical figures responded by the ten individuals were averaged was regarded as additive effects of Examples and Comparative Examples. The judgments were made such that it was recognized to have some effects when the numerical figures were from 4 to 6, and it was recognized not to have effects when the numerical figures were less than 4. The items of the sensory evaluations were "tackiness," "squeakiness," and "skin absorbability." The results are shown in Table 7.

[Table 6]

Table 6: Emulsion-type foundation

| Indicated name | Blending amount, % by mass |
| --- | --- |
| Cyclopentasiloxane | 14.2 |
| Hydrophobically treated titanium oxide | 3.6 |
| Hydrophobically treated (red) iron oxide | 0.1 |
| Hydrophobically treated (yellow) iron oxide | 0.8 |
| Hydrophobically treated (black) iron oxide | 0.1 |
| Hydrophobically treated talc | 2.0 |
| Hydrophobically treated fine titanium oxide particles | 1.2 |
| Diphenylsiloxy phenyl trimethicone | 4.5 |
| Trimethylsiloxysilicate | 0.5 |
| Methyl polymethacrylate | 2.4 |
| Distearyl dimonium hectorite | 0.7 |
| Lauryl PEG-9 polydimethylsiloxyethyl dimethicone | 1.5 |
| PEG-9 dimethicone | 2.5 |
| Ethylhexyl methoxycinnamate | 7.5 |
| Diethylaminohydroxybenzoyl hexyl benzoate | 0.5 |
| Water | Balance |
| Galactomannan degradation product (Examples, Comparative Examples) | 2.0 |
| NaCl | 1.0 |
| BG | 7.0 |
| Methylparaben | 0.1 |
| Glycerol | 3.0 |
| Total | 100.0 |

[Table 7]

Table 7

| Emulsion-type foundation | Product used | Results | | |
| --- | --- | --- | --- | --- |
| | | tackiness | squeakiness | skin absorbability |
| 1 | Comp. Ex. 1 | 1.8 | 3.8 | 2.8 |
| 2 | Ex. 3 | 5.5 | 5.4 | 5.2 |
| 3 | Ex. 4 | 5.6 | 5.8 | 5.8 |
| 4 | Without addition | 1.2 | 1.5 | 1.4 |

As is clear from the results of Table 7, when the galactomannan degradation product of Example 3 or 4 is used in an emulsion-type foundation, it can be seen that the feels such that the emulsion-type foundation does not have tackiness or squeakiness, and has favorable skin absorbability is obtained.

< Test Example 4> Nonalcoholic Beers

Each of Nonalcoholic beers 1 to 4 was prepared with a composition as listed in Table 8 using each of the galactomannan degradation products prepared in Example 3 and Comparative Example 1, Comparative Example 4 (sparingly digestible dextrin manufactured by Matsutani Chemical Industry Co., Ltd.), or Comparative Example 5 (guar gum manufactured by Dupont Corporation). In addition, the same procedures as above were carried out except that the galactomannan degradation product was not used to prepare Nonalcoholic beer 5. For each of the samples, foam-retaining property, turbidity, viscosity, and smoothness in the feel going down the throat were evaluated.

< Evaluation of Foam-Retaining Property >

Each of the nonalcoholic beers was maintained at 5°C, and 100 ml thereof was poured into a 300 ml measuring cylinder at a constant rate, and the heights of foams at 0 minutes (immediately after the rise of foams) and after 2 minutes passed were measured. The height of foams as used herein refers to a height from an interface of a liquid and foams to an upper-end part of the foams. In the present measurement, the higher the height of the foams after 2 minutes passed, it is judged to be more excellent the foam-retaining property. The results are shown in Table 9.

< Evaluation of Turbidity >

The evaluation of the turbidity was measured with a spectrophotometer. A silica cell was charged with each of the nonalcoholic beers, the absorbance with monochromatic light at 650 mm was measured, and this value was defined as turbidity. The results are shown in Table 9.

< Measurement of Viscosity >

The evaluation of the viscosity was measured with a B type viscometer manufactured by TOKI SANGYO CO., LTD. Each of the nonalcoholic beers was measured at 5°C and 250 rpm. The results are shown in Table 9.

< Smoothness in the Feel Going Down the Throat >

The sensory evaluation was conducted by 10 panelists, and a questionnaire was made on smoothness in the feel going down the throat. The panelists were asked to write down the judgments for the sensory evaluation upon drinking on the response sheet in a 6-rank evaluation, in which the numerical figure when the smoothness in the feel going down the throat is worsened was defined as 1, and the numerical figure when the smoothness in the feel going down the throat is most excellent was defined as 6. The value in which the numerical figures responded by the ten individuals were averaged was regarded as additive effects of Examples and Comparative Examples. The judgments were made such that it was recognized to have some effects when the numerical figures were from 4 to 6, and it was recognized not to have effects when the numerical figures were less than 4. The results are shown in Table 9.

[Table 8]

Table 8

| Raw materials | Blending amount, % by mass |
|---|---|
| Malt extract | 0.25 |
| Reduced maltose starch syrup | 1.2 |
| Alginate ester | 0.015 |
| Quillai saponin extract | 0.005 |
| Examples, Comparative Examples | 1 |
| Lactic acid | 0.0056 |
| Isoalpha acid | 0.006 |
| Flavor | 0.14 |

(continued)

| Raw materials | Blending amount, % by mass |
|---|---|
| Ion-exchanged water | Blended in an amount so that a total amount including the above raw materials is 20 |
| Carbonated water | 80 |
| Total | 100 |

[Table 9]

Table 9

| Nonalcoholic beer | Product used | Foam height, mm | | Turbidity, Abs. | Viscosity, mPa·s | Smoothness in the feel going down the throat |
|---|---|---|---|---|---|---|
| | | 0 min. | 2 min. | | | |
| 1 | Ex. 3 | 185 | 102 | 0.066 | 2.53 | 5.8 |
| 2 | Comp. Ex. 1 | 154 | 88 | 0.075 | 3.23 | 2.2 |
| 3 | Comp. Ex. 4 | 120 | 30 | 0.066 | 2.54 | 2.9 |
| 4 | Comp. Ex. 5 | 110 | 35 | 0.12 | 980 | 1.0 |
| 5 | Without addition | 105 | 35 | 0.066 | 2.50 | 5.8 |

As is clear from the results of Table 9, it can be seen that when the galactomannan degradation product of the present invention is used in a nonalcoholic beer, there are low influences on turbidity, viscosity, and smoothness in the feel going down the throat, so that the foam-retaining property could be improved without dropping the value as a beverage. In other words, the foam height after 2 minutes passed is higher in Nonalcoholic beer 1 in which the galactomannan degradation product of Example 3 was used, as compared to Nonalcoholic beer 5 without addition thereof, while having the same levels of turbidity, the viscosity, and the smoothness in the feel going down the throat.

INDUSTRIAL APPLICABILITY

The galactomannan degradation product of the present invention can be used in various foodstuff, cosmetics, and the like.

**Claims**

1. A galactomannan degradation product wherein a molecular weight distribution thereof satisfies the following requirements:

    a molecular weight of 12,000 or more is from 1 to 32%;
    a molecular weight of from 300 to 3,000 is from 25 to 60%; and
    a maximum of peak intensity in the molecular weight patterns of high-performance liquid chromatography exists within the range of a molecular weight of from 4,000 to 14,000.

2. The galactomannan degradation product according to claim 1, wherein the amount of component having a molecular weight of less than 300 in the molecular weight distribution is 15% or less.

3. The galactomannan degradation product according to claim 1 or 2, wherein the viscosity is 10 mPa·s or less.

4. The galactomannan degradation product according to any one of claims 1 to 3, wherein the dietary fiber content when measured according to high-performance liquid chromatography method (enzyme-HPLC method) is 50% or more.

5. Foodstuff comprising a galactomannan degradation product as defined in any one of claims 1 to 4.

6. The foodstuff according to claim 5, which is bread or emulsion-type foodstuff.

7. Cosmetics comprising a galactomannan degradation product as defined in any one of claims 1 to 4.

8. A method of improving foam property of cosmetics or foodstuff using a galactomannan degradation product as defined in any one of claims 1 to 4.

[FIG. 1]

FIG. 1

$$y = -0.3942x + 10.621$$
$$R^2 = 0.9992$$

[FIG. 2]

FIG. 2

[FIG. 3]

FIG. 3

LACTIC ACID CONTENT IN FECES

[FIG. 4]

FIG. 4

ACETIC ACID CONTENT IN FECES

[**FIG. 5**]

FIG. 5

PROPIONIC ACID CONTENT IN FECES

Control | Ex. 4 | Comp. Ex. 1 | Comp. Ex. 2 | Sparingly digestible dextrin

[**FIG. 6**]

FIG. 6

BUTYRIC ACID CONTENT IN FECES

Control | Ex. 4 | Comp. Ex. 1 | Comp. Ex. 2 | Sparingly digestible dextrin

## INTERNATIONAL SEARCH REPORT

International application No.

PCT/JP2021/007633

### A. CLASSIFICATION OF SUBJECT MATTER

C08B 37/00(2006.01)i; A23L 2/52(2006.01)i; A23L 5/00(2016.01)i; A23L 33/125(2016.01)i; A61K 8/73(2006.01)i; A61K 31/736(2006.01)i; A61P 1/14(2006.01)i; C12P19/04(2006.01)i
FI: C08B37/00; A23L5/00 N; A61K31/736; A61P1/14; A23L2/52; A23L33/125; C12P19/Q4 A; A61K8/73

According to International Patent Classification (IPC) or to both national classification and IPC

### B. FIELDS SEARCHED

Minimum documentation searched (classification system followed by classification symbols)
C08B37/00; A23L2/52; A23L5/00; A23L33/125; A61K8/73; A61K31/736; A61P1/14; C12P19/04

Documentation searched other than minimum documentation to the extent that such documents are included in the fields searched
Published examined utility model applications of Japan         1922–1996
Published unexamined utility model applications of Japan       1971–2021
Registered utility model specifications of Japan               1996–2021
Published registered utility model applications of Japan       1994–2021

Electronic data base consulted during the international search (name of data base and, where practicable, search terms used)
CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | WO 2007/122803 A1 (TAIYO KAGAKU CO., LTD.) 01 November 2007 (2007-11-01) claims, paragraphs [0001], [0011], examples 1-1 to 1-4, 3-9, paragraph [0137] | 1-6 |
| A | JP 2010-126457 A (TAIYO KAGAKU CO., LTD.) 10 June 2010 (2010-06-10) | 1-8 |
| A | JP 6-225734 A (TAIYO KAGAKU CO., LTD.) 16 August 1994 (1994-08-16) | 1-8 |
| A | WO 2000/027219 A1 (TAIYO KAGAKU CO., LTD.) 18 May 2000 (2000-05-18) | 1-8 |
| A | JP 10-036403 A (TAIYO KAGAKU CO., LTD.) 10 February 1998 (1998-02-10) | 1-8 |
| A | JP 2-248401 A (GODO SHUSEI CO., LTD.) 04 October 1990 (1990-10-04) | 1-8 |

☐ Further documents are listed in the continuation of Box C.    ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 06 May 2021 (06.05.2021) | 18 May 2021 (18.05.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT
Information on patent family members

International application no.

PCT/JP2021/007633

| Patent Documents referred in the Report | Publication Date | Patent Family | Publication Date |
|---|---|---|---|
| WO 2007/122803 A1 | 01 Nov. 2007 | JP 2007-282587 A | |
| JP 2010-126457 A | 10 Jun. 2010 | (Family: none) | |
| JP 6-225734 A | 16 Aug. 1994 | (Family: none) | |
| WO 2000/027219 A1 | 18 May 2000 | EP 1129628 A1<br>AU 9763398 A | |
| JP 10-036403 A | 10 Feb. 1998 | (Family: none) | |
| JP 2-248401 A | 04 Oct. 1990 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3008138 B
- JP 3441756 B
- JP 6247860 A
- JP HEI4346908 A